# EUROPEAN PATENT APPLICATION

(11) **EP 4 640 687 A1**
(43) Date of publication of application: **29.10.2025**
(21) Application number: 23906969.3
(22) Date of filing: 18.12.2023
(51) Int. Cl.: C07D 493/08, C07B 61/00

(54) **LINKER FOR NUCLEIC ACID SYNTHESIS, CARRIER, AND METHODS FOR PRODUCING SAME**

(30) Priority: 20.12.2022 JP 2022203402
(71) Applicant: SynCrest Inc., Fujisawa-shi, Kanagawa 251-8555 (JP)
(72) Inventor: HARI, Yoshiyuki, Tokushima-shi, Tokushima 770-8514 (JP); KAJINO, Ryohei, Fujisawa-shi, Kanagawa 251-8555 (JP)
(74) Representative: TBK
(86) International application number: PCT/JP2023/045266
(87) International publication number: WO 2024/135609

(57) **Abstract**

An object of the present invention is to newly provide a linker for nucleic acid synthesis and a carrier for solid-phase nucleic acid synthesis. Provided are a linker precursor for nucleic acid synthesis, a linker for nucleic acid synthesis, and a carrier for solid-phase nucleic acid synthesis.

## Description

### Technical Field

The present invention relates to a linker for nucleic acid synthesis, a carrier, and methods for producing the same.

### Background Art

Conventionally, in the chemical synthesis of oligonucleic acids, such as DNA and RNA, solid-phase synthesis methods using phosphoramidite compounds are used. In solid-phase synthesis using the phosphoramidite method, oligonucleic acids are extended by the following steps.

1: Deblocking step (removing protecting groups from 5'-hydroxyl groups)
2: Coupling step (condensing and extending the nucleic acid amidite in the presence of an activator)
3: Capping step (protecting unreacted hydroxyl groups with acetic anhydride etc.)
4: Oxidation or sulfurization step (reacting phosphite with an oxidizing agent or a sulfurizing agent)

After these steps are performed, oligonucleic acids are released from the solid-phase carrier into a solution using ammonia water or the like. Therefore, in the solid-phase synthesis method mentioned above, a solid-phase carrier is used to which nucleosides are bound via a linker that is cleaved under basic conditions, such as in ammonia water. As such a cleavable linker, a succinyl group is widely used, and a carrier for solid-phase nucleic acid synthesis is used in which the 3'-hydroxyl group of a nucleoside is ester-bonded to a solid-phase carrier.

In general, when using a total of 8 types of DNA (thymine: T, adenine: A, cytosine: C, and guanine: G) and RNA (uracil: U, adenine: A, cytosine: C, and guanine: G), and further modified nucleic acids, it is necessary to prepare carriers for solid-phase nucleic acid synthesis corresponding to the respective types.

Accordingly, a universal support has been developed in which a non-nucleoside linker is attached to a solid-phase carrier. The use of the universal support makes it theoretically possible to synthesize all nucleic acid sequences using a single type of solid-phase carrier.

PTL 1 and PTL 2 disclose a linker for solid-phase nucleic acid synthesis and a carrier for solid-phase nucleic acid synthesis.

PTL 3 discloses universal building blocks and supports for use in the preparation of oligomer compounds.

NPL 1 discloses cleavable spacer (CS) derivatives for tandem synthesis of multiple oligonucleotides on a single column.

### Citation List

### Patent Literature

PTL 1: JP2016-202097A
PTL 2: JP2016-204316A
PTL 3: US2004/0152905A1

### Non-patent Literature

NPL 1: Synthesis 2021; 53(23): 4440-4448

### Summary of Invention

### Technical Problem

An object of the present invention is to newly provide a linker for nucleic acid synthesis and a carrier for solid-phase nucleic acid synthesis.

### Solution to Problem

Conventionally, in solid-phase synthesis methods using universal supports, there have been problems to be considered, such as low efficiency in cleavage of oligonucleic acids from solid-phase carriers, or the production of by-products derived from non-nucleoside linkers, making it difficult to separate the by-products from the target product.

The present inventors have developed a novel universal support that can synthesize oligonucleic acids with high purity and high efficiency without producing by-products derived from non-nucleoside linkers.

That is, the present invention includes the following linker for nucleic acid synthesis, carrier for solid-phase nucleic acid synthesis, and methods for producing the same.

### Item 1.

A method for producing a linker precursor for nucleic acid synthesis represented by the following formula (2): 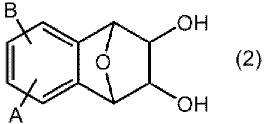 wherein
in formula (2), A is present at one substitutable position and represents a -CO-OR group, a -NH-CO-OR group, or a - CO-NH-CH₂-CO-OR group; and R represents a protecting group for carboxylic acids, and
in formula (2), B represents identically or differently, at a substitutable position, a hydrogen atom, a lower alkyl group, a lower alkoxy group, a cyano group, a nitro group, or a dialkylamino group;
the method comprising subjecting a compound represented by the following formula (1):

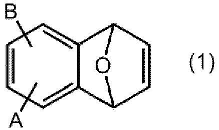

wherein in formula (1), A and B are the same as those in formula (2);
to an oxidation reaction in a solvent in the presence of an oxidizing agent and a reoxidizing agent.

### Item 2.

The production method according to Item 1, wherein the oxidizing agent is at least one oxidizing agent selected from the group consisting of osmium tetroxide (OsO₄) and ozone (O₃).

### Item 3.

The production method according to Item 1, wherein the reoxidizing agent is at least one reoxidizing agent selected from the group consisting of N-methylmorpholine-N-oxide (NMO), trimethylamine-N-oxide (Me₃NO), and tert-butyl hydroperoxide (TBHP).

### Item 4.

The production method according to Item 1, wherein the solvent is a mixed solvent of water and a hydrophilic organic solvent.

### Item 5.

A linker precursor for nucleic acid synthesis represented by the following formula (2): 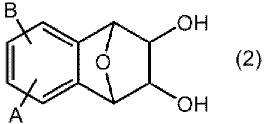 wherein
in formula (2), A is present at one substitutable position and represents a -CO-OR group, a -NH-CO-OR group, or a - CO-NH-CH₂-CO-OR group; and R represents a protecting group for carboxylic acids, and
in formula (2), B represents identically or differently, at a substitutable position, a hydrogen atom, a lower alkyl group, a lower alkoxy group, a cyano group, a nitro group, or a dialkylamino group.

### Item 6.

A method for producing a linker precursor for nucleic acid synthesis represented by the following formula (3): 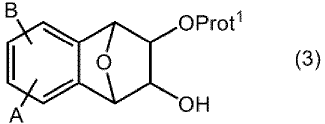 wherein
in formula (3), A is present at one substitutable position and represents a -CO-OR group, a -NH-CO-OR group, or a - CO-NH-CH₂-CO-OR group; and R represents a protecting group for carboxylic acids,
in formula (3), B represents identically or differently, at a substitutable position, a hydrogen atom, a lower alkyl group, a lower alkoxy group, a cyano group, a nitro group, or a dialkylamino group, and
in formula (3), Prot¹ represents a 4,4'-dimethoxytrityl group, a 4-methoxytrityl group, or a trityl group;
the method comprising reacting a compound represented by the following formula (2):

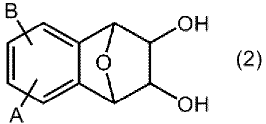

wherein in formula (2), A and B are the same as those in formula (3);
with 4,4'-dimethoxytrityl chloride (DMTr-Cl), 4-methoxytrityl chloride (MMTr-Cl), or trityl chloride (Tr-Cl) in a solvent.

### Item 7.

The production method according to Item 6, wherein the solvent is at least one organic solvent selected from the group consisting of aprotic polar solvents.

### Item 8.

A linker precursor for nucleic acid synthesis represented by the following formula (3): 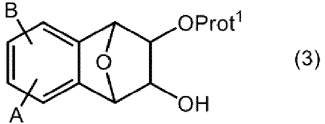 wherein
in formula (3), A is present at one substitutable position and represents a -CO-OR group, a -NH-CO-OR group, or a - CO-NH-CH₂-CO-OR group; and R represents a protecting group for carboxylic acids,
in formula (3), B represents identically or differently, at a substitutable position, a hydrogen atom, a lower alkyl group, a lower alkoxy group, a cyano group, a nitro group, or a dialkylamino group, and
in formula (3), Prot¹ represents a 4,4'-dimethoxytrityl group, a 4-methoxytrityl group, or a trityl group.

### Item 9.

A method for producing a linker for nucleic acid synthesis represented by the following formula (4): 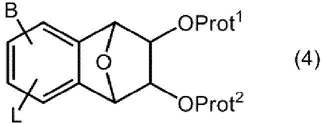 wherein
in formula (4), L is present at one substitutable position and represents a -CO-OH group, a -NH-CO-OH group, or a - CO-NH-CH₂-CO-OH group,
in formula (4), B represents identically or differently, at a substitutable position, a hydrogen atom, a lower alkyl group, a lower alkoxy group, a cyano group, a nitro group, or a dialkylamino group,
in formula (4), Prot¹ represents a 4,4'-dimethoxytrityl group, a 4-methoxytrityl group, or a trityl group, and
in formula (4), Prot² represents a protecting group for hydroxyl groups;
the method comprising subjecting a compound represented by the following formula (3): 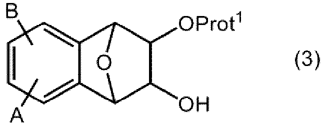 wherein
in formula (3), A is present at one substitutable position and represents a -CO-OR group, a -NH-CO-OR group, or a - CO-NH-CH₂-CO-OR group; and R represents a protecting group for carboxylic acids, and
in formula (3), B and Prot¹ are the same as those in formula (4);
to the following two steps:
(1) performing hydrolysis in a solvent, and then
(2) protecting a hydroxyl group in a solvent.

### Item 10.

The production method according to Item 9, wherein the solvent used in step (1) is at least one solvent selected from the group consisting of alkaline aqueous solutions, ether-based solvents, alcohol-based solvents, and halogen-based solvents.

### Item 11.

The production method according to Item 9, wherein the solvent used in step (2) is at least one solvent selected from the group consisting of aprotic polar solvents and ether-based solvents.

### Item 12.

A linker for nucleic acid synthesis represented by the following formula (4): 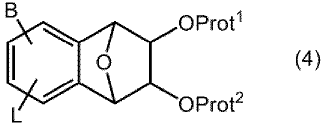 wherein
in formula (4), L is present at one substitutable position and represents a -CO-OH group, a -NH-CO-OH group, or a - CO-NH-CH₂-CO-OH group,
in formula (4), B represents identically or differently, at a substitutable position, a hydrogen atom, a lower alkyl group, a lower alkoxy group, a cyano group, a nitro group, or a dialkylamino group,
in formula (4), Prot¹ represents a 4,4'-dimethoxytrityl group, a 4-methoxytrityl group, or a trityl group, and
in formula (4), Prot² represents a protecting group for hydroxyl groups.

### Item 13.

A method for producing a carrier for solid-phase nucleic acid synthesis represented by the following formula (5): 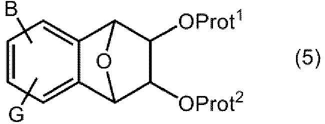 wherein
in formula (5), G is present at one substitutable position and represents the following formula (G1), (G2), or (G3),
in formula (G1), (G2), or (G3), SP represents a solid-phase carrier: 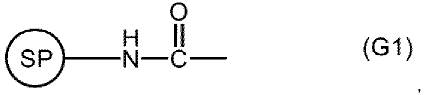 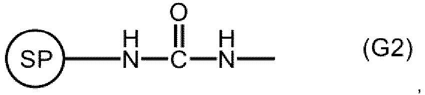 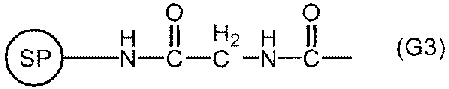
in formula (5), B represents identically or differently, at a substitutable position, a hydrogen atom, a lower alkyl group, a lower alkoxy group, a cyano group, a nitro group, or a dialkylamino group,
in formula (5), Prot¹ represents a 4,4'-dimethoxytrityl group, a 4-methoxytrityl group, or a trityl group, and
in formula (5), Prot² represents a protecting group for hydroxyl groups;
the method comprising reacting a compound represented by the following formula (4): 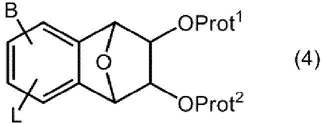 wherein
in formula (4), L is present at one substitutable position and represents a -CO-OH group, a -NH-CO-OH group, or a - CO-NH-CH₂-CO-OH group, and
in formula (4), B, Prot¹, and Prot² are the same as those in formula (5);
with a solid-phase carrier represented by the following formula: 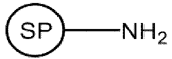
wherein SP represents a solid-phase carrier;
in a solvent in the presence of a coupling agent.

### Item 14.

The production method according to Item 13, wherein the coupling agent is at least one coupling agent selected from the group consisting of 1-[bis(dimethylamino)methylene]-1H-1,2,3-triazolo[4,5-b]pyridinium 3-oxide hexafluorophosphate (HATU), 1-[bis(dimethylamino)methylene]-1H-benzotriazolium 3-oxide hexafluorophosphate (HBTU), O-(1H-6-chlorobenzotriazole-1-yl)-1,1,3,3-tetramethyluronium hexafluorophosphate (HCTU), 1-[bis(dimethylamino)methylene]-1H-1,2,3-triazolo[4,5-b]pyridinium 3-oxide tetrafluoroborate (TATU), 1-[bis(dimethylamino)methylene]-1H-benzotriazolium 3-oxide tetrafluoroborate (TBTU), N,N'-dicyclohexylcarbodiimide (DCC), 1-ethyl-3-(3-dimethylaminopropyl)carbodiimide hydrochloride (EDC-HCl), ethyl 2-cyano-2-((dimethyliminio)(morpholino)methyloxyimino)acetate hexafluorophosphate (COMU), diphenylphosphoryl azide (DPPA), 4-(4,6-dimethoxy-1,3,5-triazin-2-yl)-4-methylmorpholinium chloride (DMT-MM), benzotriazol-1-yl-oxy-tris(dimethylamino)-phosphonium hexafluorophosphate (BOP), and 1H-benzotriazol-1-yloxytripyrrolidinophosphonium hexafluorophosphate (PyBOP).

### Item 15.

The production method according to Item 13, wherein the solvent is at least one organic solvent selected from the group consisting of aprotic polar solvents and halogen-based solvents.

### Item 16.

A carrier for solid-phase nucleic acid synthesis represented by the following formula (5): 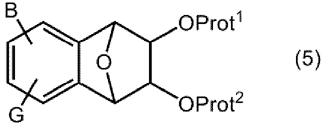 wherein
in formula (5), G is present at one substitutable position and represents the following formula (G1), (G2), or (G3),
in formula (G1), (G2), or (G3), SP represents a solid-phase carrier: 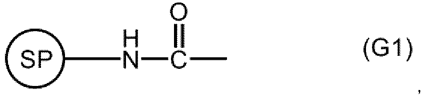 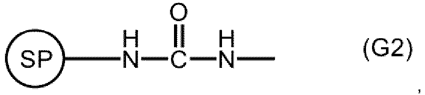 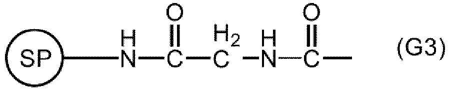
in formula (5), B represents identically or differently, at a substitutable position, a hydrogen atom, a lower alkyl group, a lower alkoxy group, a cyano group, a nitro group, or a dialkylamino group,
in formula (5), Prot¹ represents a 4,4'-dimethoxytrityl group, a 4-methoxytrityl group, or a trityl group, and
in formula (5), Prot² represents a protecting group for hydroxyl groups.

The linker for nucleic acid synthesis of the present invention can be used as a carrier for solid-phase nucleic acid synthesis.

The linker for nucleic acid synthesis of the present invention does not have a cleavable bond (ester bond) but has a non-cleavable bond (amide bond) between linker structures (between a solid-phase carrier (SP) and a linker structure) under conditions (e.g., in ammonia water) for cleaving a universal linker (a compound represented by formula (4)) from the solid-phase carrier.

The use of the linker for nucleic acid synthesis and carrier for solid-phase nucleic acid synthesis of the present invention, which have a bond (amide bond) that is not cleaved in a cleavage step under basic conditions (e.g., in ammonia water) in solid-phase nucleic acid synthesis, and which suppresses the production of by-products (linker adducts) that occur when using conventional universal linkers, makes it possible to provide high purity nucleic acids.

The linker for nucleic acid synthesis of the present invention has a bicyclic structure that promotes nucleophilic attack by the adjacent hydroxyl group on the phosphate group, thereby releasing the oligonucleic acid from the solid-phase carrier with high efficiency.

### Advantageous Effects of Invention

The present invention can newly provide a linker for nucleic acid synthesis and a carrier for solid-phase nucleic acid synthesis.

### Description of Embodiments

The present invention is described in detail below.

In the present specification, the terms "comprise" and "contain" include the concepts of "comprise," "contain," "consist essentially of," and "consist of."

In the present specification, a numerical range indicated by "A to B" means "A or more and B or less."

### [1] Method for Producing Linker Precursor for Nucleic Acid Synthesis 1

In the present invention, the method for producing a linker precursor for nucleic acid synthesis represented by the following formula (2): 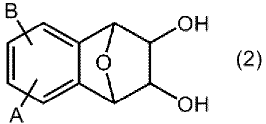 wherein
in formula (2), A is present at one substitutable position and represents a -CO-OR group, a -NH-CO-OR group, or a - CO-NH-CH₂-CO-OR group; and R represents a protecting group for carboxylic acids, and
in formula (2), B represents identically or differently, at a substitutable position, a hydrogen atom, a lower alkyl group, a lower alkoxy group, a cyano group, a nitro group, or a dialkylamino group;
comprises subjecting a compound represented by the following formula (1): 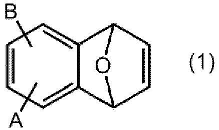
wherein in formula (1), A and B are the same as those in formula (2);
to an oxidation reaction in a solvent in the presence of an oxidizing agent and a reoxidizing agent.

### [1-1] Compound Represented by Formula (1)

The present invention comprises subjecting a compound represented by the following formula (1): 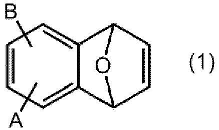 to an oxidation reaction in a solvent in the presence of an oxidizing agent and a reoxidizing agent, and produces a linker precursor for nucleic acid synthesis represented by the following formula (2):

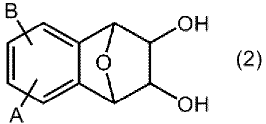

In formulas (1) and (2), A is present at one substitutable position and represents a -CO-OR group, a -NH-CO-OR group, or a -CO-NH-CH₂-CO-OR group.

R represents a protecting group for carboxylic acids. R is preferably a linear, branched, or cyclic alkyl group. Specific examples include C₁₋₄ linear or branched alkyl groups, such as methyl, ethyl, n-propyl, isopropyl, n-butyl, isobutyl, sec-butyl, tert-butyl, and 1-ethylpropyl; a benzyl group (C₆H₅CH₂-), and the like. R is more preferably a C₁₋₄ linear or branched alkyl group (a lower alkyl, even more preferably a methyl group), a benzyl group, or the like.

In formulas (1) and (2), B represents identically or differently, at a substitutable position, a hydrogen atom, a lower alkyl group, a lower alkoxy group, a cyano group, a nitro group, or a dialkylamino group.

The lower alkyl group is preferably a linear, branched, or cyclic alkyl group. Specific examples include C₁₋₄ linear or branched alkyl groups, such as methyl, ethyl, n-propyl, isopropyl, n-butyl, isobutyl, sec-butyl, tert-butyl, and 1-ethylpropyl.

The lower alkoxy group is preferably a C₁₋₄ linear or branched alkoxy group, such as methoxy, ethoxy, n-propoxy, isopropyloxy, n-butoxy, isobutyloxy, sec-butoxy, tert-butoxy, or 1-ethylpropyloxy. The lower alkoxy group is preferably methoxy or tert-butoxy.

The dialkylamino group is preferably a dialkylamino group having two linear or branched alkyl groups having about 1 or 2 carbon atoms, such as a dimethylamino group, a diethylamino group, or an ethylmethylamino group.

### [1-2] Solvent

In the production method of the present invention, the solvent is preferably a mixed solvent of water and a hydrophilic organic solvent.

The hydrophilic organic solvent is preferably a hydrophilic organic solvent, such as an aprotic polar solvent, an ether-based solvent, or an ester-based solvent.

The aprotic polar solvent is preferably acetone, acetonitrile (CH₃CN), N,N-dimethylformamide (DMF), dimethyl sulfoxide (DMSO), pyridine, or the like.

The ether-based solvent is preferably tetrahydrofuran (THF), dioxane, diethyl ether (Et₂O), isopropyl ether (IPE), or the like.

The ester-based solvent is preferably ethyl acetate (AcOEt) or the like.

The hydrophilic organic solvent is more preferably an aprotic polar solvent, such as acetone, acetonitrile, DMF, or DMSO; or an ether-based solvent (polar solvent), such as THF, dioxane, or diethyl ether.

The mixing ratio of water to the hydrophilic organic solvent is preferably such that the volume ratio of water to the hydrophilic organic solvent is 3:7 to 9:1, more preferably 4:6 to 8:2, and even more preferably 5:5 to 7:3.

The hydrophilic organic solvent is even more preferably a mixed solvent of water and acetonitrile.

The amount of the hydrophilic solvent used is adjusted so that the concentration (molar concentration M (mol/L)) of the compound represented by formula (1) (substrate) is preferably 0.01 M to 5 M, and more preferably 0.1 M to 2 M.

### [1-3] Oxidizing Agent

In the production method of the present invention, the oxidizing agent is preferably at least one oxidizing agent selected from the group consisting of osmium tetroxide (OsO₄) and ozone (O₃).

In the production method of the present invention, an oxidizing agent is used to promote the oxidation reaction, adding a hydroxyl group to the compound represented by formula (1) (substrate).

The amount of the oxidizing agent used (oxidation reaction) is adjusted so that the concentration (molar concentration M (mol/L)) in the solvent is preferably 0.01 M to 5 M, and more preferably 0.1 M to 2 M.

The amount of the oxidizing agent used (oxidation reaction) is adjusted so that the concentration relative to the compound represented by formula (1) (substrate) (when the amount of the substrate is taken as 100 mol%) is preferably 0.01 mol% to 100 mol%, more preferably 0.1 mol% to 50 mol%, and even more preferably 1 mol% to 10 mol%, in terms of molar ratio.

### [1-4] Reoxidizing Agent

In the production method of the present invention, the reoxidizing agent is preferably at least one reoxidizing agent selected from the group consisting of N-methylmorpholine-N-oxide (NMO), trimethylamine-N-oxide (Me₃NO), and tert-butyl hydroperoxide (TBHP).

In the production method of the present invention, a reoxidizing agent is used to promote the oxidation reaction, adding a hydroxyl group to the compound represented by formula (1) (substrate).

The amount of the reoxidizing agent used (oxidation reaction) is adjusted so that the concentration (molar concentration M (mol/L)) in the solvent is preferably 0.01 M to 5 M, and more preferably 0.1 M to 2 M.

The amount of the reoxidizing agent used (oxidation reaction) is adjusted so that the concentration relative to the compound represented by formula (1) (substrate) (when the amount of the substrate is taken as 100 mol%) is preferably 50 mol% to 10,000 mol%, more preferably 90 mol% to 1000 mol%, and even more preferably 100 mol% to 500 mol%, in terms of molar ratio.

### [1-5] Oxidation Reaction Step

The reaction temperature of the oxidation reaction is preferably 0°C to 100°C, more preferably 10°C to 80°C, and even more preferably 20°C to 60°C.

The reaction time of the oxidation reaction is preferably 0.1 hours to 100 hours, more preferably 0.1 hours to 50 hours, and even more preferably 0.1 hours to 10 hours.

### [1-6] Linker Precursor for Nucleic Acid Synthesis 1 Represented by Formula (2)

The present invention includes a linker precursor for nucleic acid synthesis represented by the following formula (2):

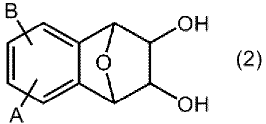

In formula (2), A and B are as described above.

### [2] Method for Producing Linker Precursor for Nucleic Acid Synthesis 2

In the present invention, the method for producing a linker precursor for nucleic acid synthesis represented by the following formula (3): 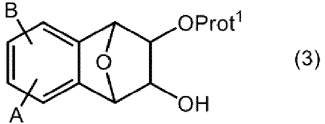 wherein
in formula (3), A is present at one substitutable position and represents a -CO-OR group, a -NH-CO-OR group, or a - CO-NH-CH₂-CO-OR group; and R represents a protecting group for carboxylic acids,
in formula (3), B represents identically or differently, at a substitutable position, a hydrogen atom, a lower alkyl group, a lower alkoxy group, a cyano group, a nitro group, or a dialkylamino group, and
in formula (3), Prot¹ represents a 4,4'-dimethoxytrityl group, a 4-methoxytrityl group, or a trityl group;
comprises reacting a compound represented by the following formula (2): 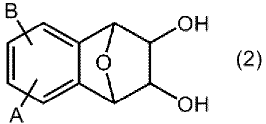
wherein in formula (2), A and B are the same as those in formula (3);
with 4,4'-dimethoxytrityl chloride (DMTr-Cl), 4-methoxytrityl chloride (MMTr-Cl), or trityl chloride (Tr-Cl) in a solvent.

### [2-1] Compound Represented by Formula (2)

The present invention comprises reacting a compound represented by the following formula (2): 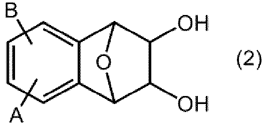 with 4,4'-dimethoxytrityl chloride (DMTr-Cl), 4-methoxytrityl chloride (MMTr-Cl), or trityl chloride (Tr-Cl) in a solvent, and produces a linker precursor for nucleic acid synthesis represented by the following formula (3):

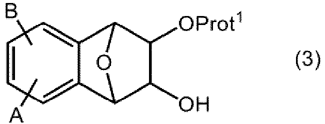

In formula (2), A and B are as described above.

In formula (3), A and B are the same as those in formula (2).

In formula (3), Prot¹ represents a 4,4'-dimethoxytrityl group (DMTr group), a 4-methoxytrityl group (MMTr group), or a trityl group (Tr group).

### [2-2] Solvent

In the production method of the present invention, the solvent is preferably at least one organic solvent selected from the group consisting of aprotic polar solvents.

The aprotic polar solvents are more preferably acetone, acetonitrile (CH₃CN), N,N-dimethylformamide (DMF), dimethyl sulfoxide (DMSO), pyridine, tetrahydrofuran (THF), and the like.

The organic solvent is even more preferably pyridine.

The amount of the solvent used is adjusted so that the concentration (molar concentration M (mol/L)) of the compound represented by formula (2) (substrate) is preferably 0.01 M to 5 M, and more preferably 0.1 M to 2 M.

### [2-3] 4,4'-Dimethoxytrityl Chloride (DMTr-Cl), 4-Methoxytrityl Chloride (MMTr-Cl), or Trityl Chloride (Tr-Cl)

In the production method of the present invention, DMTr-Cl, MMTr-Cl, or Tr-Cl is used to protect the hydroxyl group of the compound represented by formula (2) (substrate).

The amount of DMTr-Cl, MMTr-Cl, or Tr-Cl used (hydroxyl group protection reaction) is adjusted so that the concentration (molar concentration M (mol/L)) in the solvent is preferably 0.01 M to 5 M, and more preferably 0.1 M to 2 M.

The amount of DMTr-Cl, MMTr-Cl, or Tr-Cl used (hydroxyl group protection reaction) is adjusted so that the concentration relative to the compound represented by formula (2) (substrate) (when the amount of the substrate is taken as 100 mol%) is preferably 0.01 mol% to 100 mol%, more preferably 0.1 mol% to 50 mol%, and even more preferably 1 mol% to 10 mol%, in terms of molar ratio.

### [2-4] Hydroxyl Group Protection Reaction Step

The reaction temperature of the hydroxyl group protection reaction is preferably 0°C to 100°C, more preferably 10°C to 80°C, and even more preferably 20°C to 60°C.

The reaction time of the hydroxyl group protection reaction is preferably 0.1 hours to 100 hours, more preferably 0.1 hours to 50 hours, and even more preferably 0.1 hours to 20 hours.

### [2-5] Linker Precursor for Nucleic Acid Synthesis 2

The present invention includes a linker precursor for nucleic acid synthesis represented by the following formula (3):

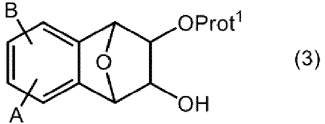

In formula (3), A and B are the same as those in formula (2).

In formula (3), Prot¹ represents a 4,4'-dimethoxytrityl group (DMTr group), a 4-methoxytrityl group (MMTr group), or a trityl group (Tr group).

In formula (3), of Prot¹, the DMTr group is derived from DMTr-Cl used in the hydroxyl group protection reaction.

In formula (3), of Prot¹, the MMTr group is derived from MMTr-Cl used in the hydroxyl group protection reaction.

In formula (3), of Prot¹, the Tr group is derived from Tr-Cl used in the hydroxyl group protection reaction.

### [3] Method for Producing Linker for Nucleic Acid Synthesis

In the present invention, the method for producing a linker for nucleic acid synthesis represented by the following formula (4): 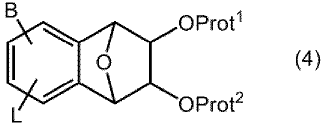 wherein
in formula (4), L is present at one substitutable position and represents a -CO-OH group, a -NH-CO-OH group, or a - CO-NH-CH₂-CO-OH group,
in formula (4), B represents identically or differently, at a substitutable position, a hydrogen atom, a lower alkyl group, a lower alkoxy group, a cyano group, a nitro group, or a dialkylamino group,
in formula (4), Prot¹ represents a 4,4'-dimethoxytrityl group, a 4-methoxytrityl group, or a trityl group, and
in formula (4), Prot² represents a protecting group for hydroxyl groups;
comprises subjecting a compound represented by the following formula (3): 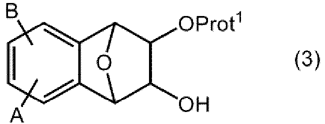 wherein
in formula (3), A is present at one substitutable position and represents a -CO-OR group, a -NH-CO-OR group, or a - CO-NH-CH₂-CO-OR group; and R represents a protecting group for carboxylic acids, and
in formula (3), B and Prot¹ are the same as those in formula (4);
to the following 2 steps:
(1) performing hydrolysis in a solvent, and then
(2) protecting a hydroxyl group in a solvent.

### [3-1] Compound Represented by Formula (3)

The present invention comprises subjecting a compound represented by the following formula (3): 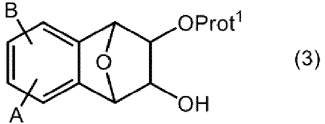 to the following 2 steps:
(1) performing hydrolysis in a solvent, and then
(2) protecting a hydroxyl group in a solvent;
and produces a linker for nucleic acid synthesis represented by the following formula (4):

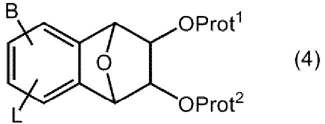

In formula (3), A, B, and Prot¹ are as described above.

In formula (4), B and Prot¹ are the same as those in formula (3).

In formula (4), L is present at one substitutable position and represents a -CO-OH group, a -NH-CO-OH group, or a - CO-NH-CH₂-CO-OH group.

In formula (4), Prot² represents a protecting group for hydroxyl groups.

### [3-2] (1) Hydrolyzing Compound Represented by Formula (3) (Substrate) in Solvent

In the production method of the present invention, (1) the compound represented by formula (3) (substrate) is subjected to a hydrolysis reaction in a solvent to hydrolyze A (a -CO-OR group, a -NH-CO-OR group, or a -CO-NH-CH₂-CO-OR group; R: a protecting group for carboxylic acids) in formula (3) to L (a - CO-OH group, a -NH-CO-OH group, or a -CO-NH-CH₂-CO-OH group) in formula (4).

### Solvent

In the production method of the present invention, the solvent used in step (1) is preferably at least one solvent selected from the group consisting of alkaline aqueous solutions, ether-based solvents, alcohol-based solvents, and halogen-based solvents.

The alkaline aqueous solutions are preferably a sodium hydroxide aqueous solution, a sodium carbonate aqueous solution, a calcium hydroxide aqueous solution, a potassium hydroxide aqueous solution, ammonia water, a sodium bicarbonate aqueous solution, and the like.

The ether-based solvents are preferably tetrahydrofuran (THF), dioxane, diethyl ether (Et₂O), isopropyl ether (IPE), and the like.

The alcohol-based solvents are preferably methanol (CH₃OH), ethanol, propanol, isopropanol, hexafluoroisopropanol (HFIP), trifluoromethanol, pentafluoroethanol, and the like.

The halogen-based solvents are preferably dichloromethane (DCM, CH₂Cl₂), trichloromethane (CHCl₃), and the like.

The amount of the solvent used is adjusted so that the concentration (molar concentration M (mol/L)) of the compound represented by formula (3) (substrate) is preferably 0.01 M to 5 M, and more preferably 0.1 M to 2 M.

### Hydrolysis Reaction

The reaction temperature of the hydrolysis reaction is preferably 0°C to 100°C, more preferably 10°C to 80°C, and even more preferably 20°C to 60°C.

The reaction time of the hydrolysis reaction is preferably 0.1 hours to 100 hours, more preferably 0.1 hours to 50 hours, and even more preferably 0.1 hours to 10 hours.

### [3-3] (2) Protecting Hydroxyl Group in Solvent after Step (1)

In the production method of the present invention, after step (1), (2) a reaction for protecting the hydroxyl group of the compound represented by formula (3) (substrate) is carried out in a solvent to protect the hydroxyl group (-OH) in formula (3) with Prot² (a protecting group for hydroxyl groups, such as an Ac group) in formula (4).

### Solvent

In the production method of the present invention, the solvent used in step (2) is preferably at least one solvent selected from the group consisting of aprotic polar solvents and ether-based solvents.

The aprotic polar solvents are preferably acetone, acetonitrile, N,N-dimethylformamide (DMF), dimethyl sulfoxide (DMSO), pyridine, and the like.

The ether-based solvents are preferably tetrahydrofuran (THF), dioxane, diethyl ether (Et₂O), isopropyl ether (IPE), and the like.

The amount of the solvent used is adjusted so that the concentration (molar concentration M (mol/L)) of the compound represented by formula (3) (substrate) is preferably 0.01 M to 5 M, and more preferably 0.1 M to 2 M.

### Hydroxyl Group Protection Reagent

For the reaction to protect the hydroxyl group, acetic anhydride (Ac₂O, Ac group protection), pivaloyl chloride (PvCl, Pv group protection), p-toluenesulfonyl isocyanate (tosyl isocyanate) (PTSI, p-toluenesulfonylcarbamate group protection), or the like is used.

The amount of Ac₂O, PvCl, PTSI, or the like used (hydroxyl group protection reaction) is adjusted so that the concentration (molar concentration M (mol/L)) in the solvent is preferably 0.01 M to 5 M, and more preferably 0.1 M to 2 M.

The amount of Ac₂O, PvCl, PTSI, or the like used (hydroxyl group protection reaction) is adjusted so that the concentration relative to the compound represented by formula (3) (substrate) (when the amount of the substrate is taken as 100 mol%) is preferably 0.01 mol% to 100 mol%, more preferably 0.1 mol% to 50 mol%, and even more preferably 1 mol% to 10 mol%, in terms of molar ratio.

### Hydroxyl Group Protection Reaction

The reaction temperature of the hydroxyl group protection reaction is preferably 0°C to 100°C, more preferably 10°C to 80°C, and even more preferably 20°C to 60°C.

The reaction time of the hydroxyl group protection reaction is preferably 0.1 hours to 100 hours, more preferably 0.1 hours to 50 hours, and even more preferably 0.1 hours to 30 hours.

### [3-4] Linker for Nucleic Acid Synthesis

The present invention includes a linker for nucleic acid synthesis represented by the following formula (4):

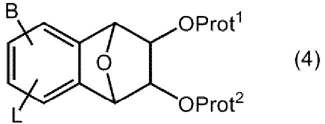

In formula (4), B and Prot¹ are the same as those in formula (3).

In formula (4), L is present at one substitutable position and represents a -CO-OH group, a -NH-CO-OH group, or a - CO-NH-CH₂-CO-OH group.

In formula (4), Prot² represents a protecting group for hydroxyl groups.

The protecting group for hydroxyl groups is preferably a protecting group that can be deprotected under alkaline (basic) conditions, and is an acyl-based protecting group. The protecting group for hydroxyl groups is more preferably an acetyl group (Ac group), a pivaloyl group (Pv group), a p-toluenesulfonylcarbamate group, or the like.

In formula (4), when Prot² is an Ac group, it is derived from acetic anhydride (Ac₂O) used in the hydroxyl group protection reaction.

In formula (4), when Prot² is a Pv group, it is derived from pivaloyl chloride (PvCl) used in the hydroxyl group protection reaction.

In formula (4), when Prot² is a p-toluenesulfonylcarbamate group, it is derived from p-toluenesulfonyl isocyanate (PTSI) used in the hydroxyl group protection reaction.

### [4] Method for Producing Carrier for Solid-Phase Nucleic Acid Synthesis

In the present invention, the method for producing a carrier for solid-phase nucleic acid synthesis represented by the following formula (5): 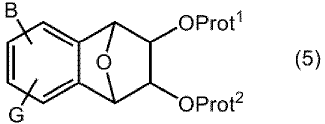 wherein
in formula (5), G is present at one substitutable position and represents the following formula (G1), (G2), or (G3),
in formula (G1), (G2), or (G3), SP represents a solid-phase carrier, 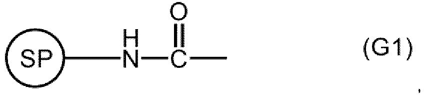 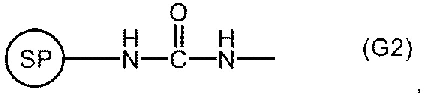 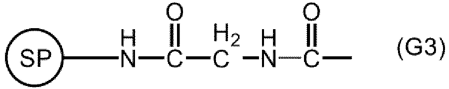
in formula (4), B represents identically or differently, at a substitutable position, a hydrogen atom, a lower alkyl group, a lower alkoxy group, a cyano group, a nitro group, or a dialkylamino group,
in formula (4), Prot¹ represents a 4,4'-dimethoxytrityl group, a 4-methoxytrityl group, or a trityl group, and
in formula (4), Prot² represents a protecting group for hydroxyl groups;
comprises reacting a compound represented by the following formula (4): 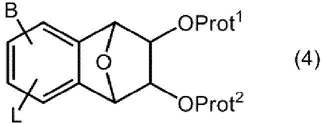 wherein
in formula (4), L is present at one substitutable position and represents a -CO-OH group, a -NH-CO-OH group, or a - CO-NH-CH₂-CO-OH group, and
in formula (4), B, Prot¹, and Prot² are the same as those in formula (5);
with a solid-phase carrier represented by the following formula: 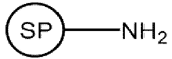
wherein SP represents a solid-phase carrier;
in a solvent in the presence of a coupling agent.

### [4-1] Compound Represented by Formula (4)

The present invention comprises reacting a compound represented by the following formula (4): 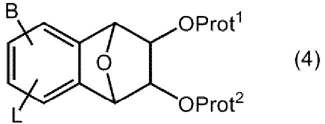 with a solid-phase carrier represented by the following formula: 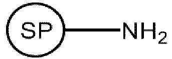 in a solvent in the presence of a coupling agent, and produces a carrier for solid-phase nucleic acid synthesis represented by the following formula (5):

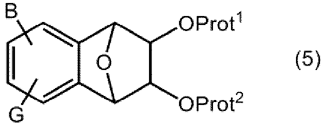

In formula (4), B, L, Prot¹, and Prot² are as described above.

In formula (5), B, Prot¹, and Prot² are the same as those in formula (4).

SP represents a solid-phase carrier.

In formula (5), G is present at one substitutable position and represents the following formula (G1), (G2), or (G3). In formula (G1), (G2), or (G3), SP represents a solid-phase carrier.

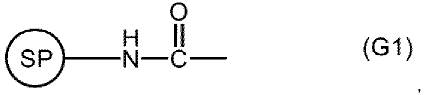

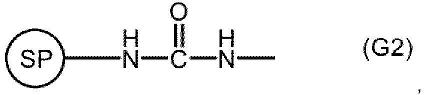

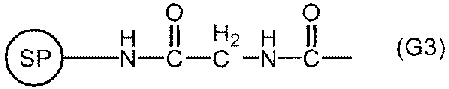

### [4-2] Solvent

In the production method of the present invention, the solvent is preferably at least one organic solvent selected from the group consisting of aprotic polar solvents and halogen-based solvents.

The aprotic polar solvents are preferably N,N-diisopropylethylamine (DIPEA), acetonitrile (ACN), acetone, N,N-dimethylformamide (DMF), dimethyl sulfoxide (DMSO), pyridine, and the like.

The halogen-based solvents are preferably dichloromethane (CH₂Cl₂), trichloromethane (CHCl₃), and the like.

The amount of the solvent used is adjusted so that the concentration (molar concentration M (mol/L)) of the compound represented by formula (4) (substrate) is preferably 0.01 M to 5 M, and more preferably 0.1 M to 2 M.

### [4-3] Coupling Agent

In the production method of the present invention, a coupling agent is used to promote the coupling reaction, reacting the compound represented by formula (4) with a solid-phase carrier (SP-NH₂) to produce a carrier for solid-phase nucleic acid synthesis represented by formula (5).

In the production method of the present invention, the coupling agent is preferably at least one coupling agent selected from the group consisting of 1-[bis(dimethylamino)methylene]-1H-1,2,3-triazolo[4,5-b]pyridinium 3-oxide hexafluorophosphate (HATU), 1-[bis(dimethylamino)methylene]-1H-benzotriazolium 3-oxide hexafluorophosphate (HBTU), O-(1H-6-chlorobenzotriazole-1-yl)-1,1,3,3-tetramethyluronium hexafluorophosphate (HCTU), 1-[bis(dimethylamino)methylene]-1H-1,2,3-triazolo[4,5-b]pyridinium 3-oxide tetrafluoroborate (TATU), 1-[bis(dimethylamino)methylene]-1H-benzotriazolium 3-oxide tetrafluoroborate (TBTU), N,N'-dicyclohexylcarbodiimide (DCC), 1-ethyl-3-(3-dimethylaminopropyl)carbodiimide hydrochloride (EDC-HCl), ethyl 2-cyano-2-((dimethyliminio)(morpholino)methyloxyimino)acetate hexafluorophosphate (COMU), diphenylphosphoryl azide (DPPA), 4-(4,6-dimethoxy-1,3,5-triazin-2-yl)-4-methylmorpholinium chloride (DMT-MM), benzotriazol-1-yl-oxy-tris(dimethylamino)-phosphonium hexafluorophosphate (BOP), and 1H-benzotriazol-1-yloxytripyrrolidinophosphonium hexafluorophosphate (PyBOP).

The amount of the coupling agent used (coupling reaction) is adjusted so that the concentration (molar concentration M (mol/L)) in the solvent is preferably 0.01 M to 5 M, and more preferably 0.1 M to 2 M.

The amount of the coupling agent (coupling agent reaction) is adjusted so that the concentration relative to the compound represented by formula (4) (substrate) (when the amount of the substrate is taken as 100 mol%) is preferably 50 mol% to 10,000 mol%, more preferably 90 mol% to 1000 mol%, and even more preferably 100 mol% to 500 mol%, in terms of molar ratio.

### [4-4] Coupling Reaction Step

In the production method of the present invention, a compound represented by formula (4) (substrate) is coupled to a solid-phase carrier in a solvent in the presence of a coupling agent, whereby the compound represented by formula (4) (substrate) and the solid-phase carrier (SP-NH₂) are bonded via an amide bond (a bond that is not cleaved under basic conditions) to produce a carrier for solid-phase nucleic acid synthesis represented by formula (5).

The carrier for solid-phase nucleic acid synthesis represented by formula (5) of the present invention can suppress the production of by-products (linker adducts) by binding a solid-phase carrier (SP) and a universal linker (the compound represented by formula (4)) via a bond (e.g., an amide bond) that is not cleaved under cleavage conditions (e.g., in ammonia water).

The reaction temperature of the coupling reaction is preferably 0°C to 100°C, more preferably 10°C to 80°C, and even more preferably 20°C to 60°C.

The reaction time of the coupling reaction is preferably 0.1 hours to 100 hours, more preferably 0.1 hours to 50 hours, and even more preferably 0.1 hours to 10 hours.

### [4-5] Carrier for Solid-Phase Nucleic Acid Synthesis

The present invention includes a carrier for solid-phase nucleic acid synthesis represented by the following formula (5):

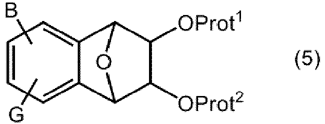

In formula (5), B, Prot¹, and Prot² are the same as those in formula (4).

In formula (5), G is present at one substitutable position and represents the following formula (G1), (G2), or (G3). In formula (G1), (G2), or (G3), SP represents a solid-phase carrier.

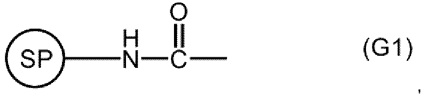

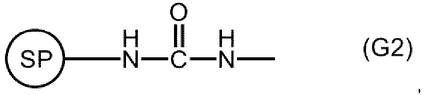

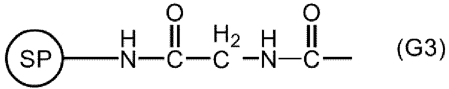

Conventionally, a universal support has been developed that is theoretically capable of synthesizing all nucleic acid sequences using a single type of solid-phase carrier. However, conventional universal supports have non-nucleoside linkers attached to solid-phase carriers, and there are some areas that need to be improved, such as the production of by-products derived from the non-nucleoside linkers, the low efficiency of cleavage of oligonucleic acids from the solid-phase carrier, and the difficulty of separating the produced by-products from the target product (full-length product).

The present invention relates to a linker for nucleic acid synthesis and a carrier for solid-phase nucleic acid synthesis, both of which can suppress the production of by-products (linker adducts) that occur when using conventional universal supports.

The linker for nucleic acid synthesis and carrier for solid-phase nucleic acid synthesis of the present invention are characterized in that the solid-phase carrier and the linker for nucleic acid synthesis are bonded via a bond (e.g., an amide bond) that is not cleaved under cleavage conditions, such as in ammonia water. The linker for nucleic acid synthesis and carrier for solid-phase nucleic acid synthesis of the present invention do not produce linker adducts, and highly pure oligonucleic acids can be obtained by using the linker for nucleic acid synthesis and carrier for solid-phase nucleic acid synthesis of the present invention.

The embodiments of the present invention are described above; however, the present invention is not limited to these examples. Needless to say, the present invention can be implemented in various forms within the scope not departing from the gist of the present invention.

### Examples

Embodiments of the present invention are described in more detail below based on Examples.

The present invention is not limited thereto.

### [1] Production of Linker Precursor for Nucleic Acid Synthesis 1

The linker precursor for nucleic acid synthesis 1 represented by formula (2) of the present invention was produced.

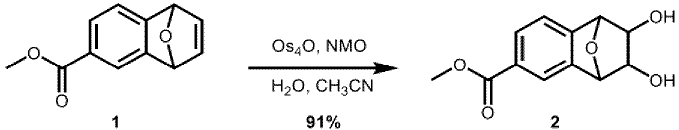

### Production of Compound 2

A 0.1M osmium tetroxide (Os₄O)/t-butyl alcohol solution (50.94 µL, 5.094 µmol) and a 50% N-methylmorpholine oxide (NMO) aqueous solution (2.65 mL, 12.735 mmol) were added to an acetonitrile (CH₃CN) solution (10 mL) of compound 1 (1.03 g, 5.09 mmol), followed by stirring at 40°C for 10 minutes.

Then, 0.5 mL of a saturated sodium thiosulfate aqueous solution was added to stop the reaction. After dilution with ethyl acetate, the organic layer was washed with distilled water and saturated saline.

After dehydration with sodium sulfate, the solvent was removed under reduced pressure, thereby obtaining compound 2 (1.09 g, 4.61 mmol, 91%) as a white solid.

1H NMR (500 MHz, DMSO-d₆) δ 7.90 (s, 1H), 7.83 (dd, J=7.5, 1.5 Hz, 1H), 7.48 (d, J=7.5 Hz, 1H), 5.13 (d, J=13.0 Hz, 2H), 5.07 (t, J=5.0 Hz, 2H), 3.84 (s, 3H), 3.74-3.70 (m, 2H).

### [2] Production of Linker Precursor for Nucleic Acid Synthesis 2

The linker precursor for nucleic acid synthesis 2 represented by formula (3) of the present invention was produced.

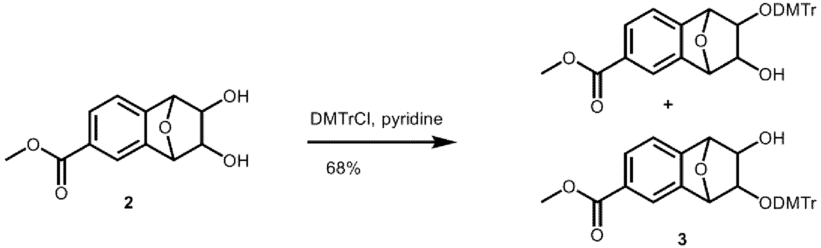

### Production of Compound 3

4,4'-dimethoxytrityl chloride (DMTrCl) (193 mg, 0.570 mmol) was added to a pyridine solution (4 mL) of compound 2 (112 mg, 0.475 mmol), followed by stirring at room temperature for 20 hours.

After dilution with ethyl acetate, the organic layer was washed with distilled water and saturated saline.

After dehydration with sodium sulfate, the solvent was removed under reduced pressure.

Then, the resulting residue was purified by silica gel column chromatography (hexane/ethyl acetate/triethylamine = 80/20/1), thereby obtaining compound 3 (173 mg, 0.322 mmol, 68%) as a yellow solid.

1H NMR (500 MHz, CDCl₃) δ 7.83-7.78 (m, 3H), 7.47-7.42 (m, 5H), 7.37-7.33 (m, 12H), 7.30-7.29 (m, 2H), 6.92-6.88 (m, 8H), 5.18 (d, J=5.5 Hz, 2H), 3.95-3.92 (m, 2H), 3.89 (s, 3H), 3.87 (s, 3H), 3.84-3.79 (m, 14H), 3.74 (dd, J=7.5, 4.0 Hz, 2H).

### [3] Production of Linker for Nucleic Acid Synthesis (2 Steps)

The linker for nucleic acid synthesis represented by formula (4) of the present invention was produced.

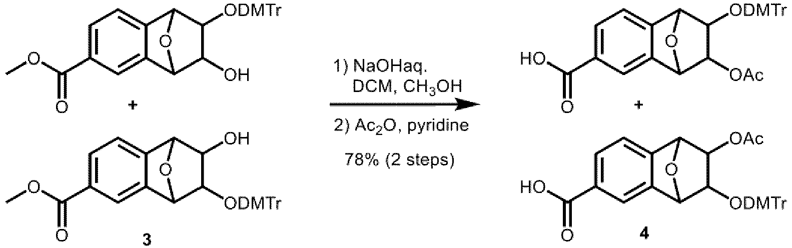

### Production of Compound 4

### (1) Step of Performing Hydrolysis in Solvent

Methanol (CH₃OH) (0.54 mL) and a 1M sodium hydroxide aqueous solution (NaOH aq) (0.20 mL) were added to a dichloromethane (DCM) solution (0.54 mL) of compound 3 (54 mg, 0.10 mmol), followed by stirring at 45°C for 2.5 hours.

Then, a 25% acetic acid aqueous solution (50 µL) was added to neutralize the reaction liquid.

After dilution with ethyl acetate, the organic layer was washed with saturated saline.

After dehydration with sodium sulfate, the solvent was removed under reduced pressure.

### (2) Step of Protecting Hydroxyl Group in Solvent

Then, the resulting residue was dissolved in pyridine (1.0 mL), and 4-dimethylaminopyridine (11.2 mg) and acetic anhydride (Ac₂O) (86 µL) were added thereto, followed by stirring at room temperature for 23 hours.

After dilution with ethyl acetate, the organic layer was washed with distilled water and saturated saline.

After dehydration with sodium sulfate, the solvent was removed under reduced pressure.

Then, the resulting residue was purified by silica gel column chromatography (dichloromethane/methanol = 60/1), thereby obtaining compound 4 (40.7 mg, 0.072 mmol, 78%) as a yellow solid.

1H NMR (500 MHz, CDCl₃) δ 7.93 (s, 1H), 7.91-7.86 (m, 2H), 7.51-7.47 (m, 5H), 7.40-7.31 (m, 14H), 6.91-6.86 (m, 10H), 5.26 (d, J=7.0 Hz, 2H), 5.09 (dd, J=11.0, 6.0 Hz, 2H), 3.92 (dd, J=6.0, 1.5 Hz, 2H), 3.83-3.78 (m, 14H).

### [4] Production 1 of Carrier for Solid-Phase Nucleic Acid Synthesis

The carrier for solid-phase nucleic acid synthesis represented by formula (5) of the present invention was produced.

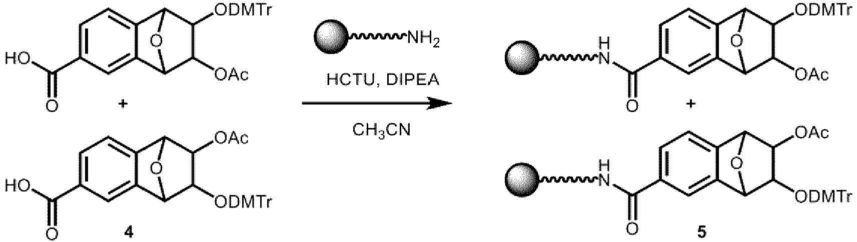

### Production of Carrier for Solid-Phase Nucleic Acid Synthesis 5-1

Compound 4 (9.5 mg, 16.8 µmol), O-(1H-6-chlorobenzotriazole-1-yl)-1,1,3,3-tetramethyluronium hexafluorophosphate (HCTU) (7.0 mg, 16.8 µmol), and N,N-diisopropylethylamine (DIPEA) (5.9 µL, 33.6 µmol) were dissolved in acetonitrile (CH₃CN) (1 mL).

Then, the mixed solution was added to an amino group-containing CPG solid-phase carrier (105 µmol/g, 200 mg, amino group: 21 µmol), followed by shaking for 3 hours.

Then, the reaction solution was filtered by suction, and the solid-phase carrier was washed with acetonitrile.

Then, 1 mL of CapMixA (acetic anhydride/pyridine/tetrahydrofuran = 10/10/80) and 1 mL of CapMixB (16% 1-methylimidazole/tetrahydrofuran solution) were used to protect unreacted amino groups with acetyl groups.

After the reaction solution was filtered by suction, the solid-phase carrier was washed with acetonitrile and dried under reduced pressure.

Then, the resulting carrier for solid-phase nucleic acid synthesis 5-1 was treated with a deblocking solution (3% trichloroacetic acid/dichloromethane solution), after which the absorbance of the generated trithyl cation was measured to measure the amount of compound 4 carried (n = 3).

### Chemical Structure of Carrier for Solid-Phase Nucleic Acid Synthesis 5-1

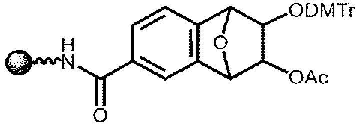

**Table 1**

| Table 1 | | | | |
|---|---|---|---|---|
| | Resin amount | Abs504 | Solution amount | |
| 1st time | 2.1 mg | 0.433 | 20 mL → | 54.3 µmol/g |
| 2nd time | 2.0 mg | 0.318 | 20 mL → | 41.8 µmol/g |
| 3rd time | 2.4 mg | 0.413 | 20 mL → | 45.3 µmol/g |
| Average | | | | 47.1 µmol/g |

The measurement results showed that the amount of compound 4 carried in the resulting carrier for solid-phase nucleic acid synthesis 5 was 47.1 µmol/g.

### Production of Carrier for Solid-Phase Nucleic Acid Synthesis 5-2

Fmoc-protected glycine (47 mg, 158 µmol), HCTU (59 mg, 189 µmol), and N,N-diisopropylethylamine (55 µL, 420 µmol) were dissolved in N,N'-dimethylformamide (1 mL).

Then, the mixed solution was added to an amino group-containing CPG solid-phase carrier (105 µmol/g, 150 mg, amino group: 15.8 µmol), followed by shaking for 2 hours.

Then, the reaction solution was filtered by suction, and the solid-phase carrier was washed with acetonitrile.

Then, 1 mL of CapMixA (acetic anhydride/pyridine/tetrahydrofuran = 10/10/80) and 1 mL of CapMixB (16% 1-methylimidazole/tetrahydrofuran solution) were used to protect unreacted amino groups with acetyl groups.

After the reaction solution was filtered by suction, the solid-phase carrier was washed with acetonitrile and dried under reduced pressure.

Then, the resulting solid-phase carrier carrying glycine was used to produce a carrier for solid-phase nucleic acid synthesis 5-2 carrying compound 4 in the same manner as for the carrier for solid-phase nucleic acid synthesis 5-1.

Then, the resulting carrier for solid-phase nucleic acid synthesis 5-2 was treated with a deblocking solution (3% trichloroacetic acid/dichloromethane solution), after which the absorbance of the generated trithyl cation was measured to measure the amount of compound 4 carried (n = 3).

### Chemical Structure of Carrier for Solid-Phase Nucleic Acid Synthesis 5-2

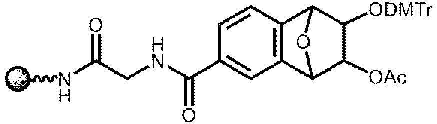

**Table 2**

| Table 2 | | | | |
|---|---|---|---|---|
| | Resin amount | Abs504 | Solution amount | |
| 1st time | 2.1 mg | 0.268 | 20 mL → | 33.6 µmol/g |
| 2nd time | 1.9 mg | 0.276 | 20 mL → | 38.2 µmol/g |
| 3rd time | 1.9 mg | 0.293 | 20 mL → | 40.6 µmol/g |
| Average | | | | 37.5 µmol/g |

The measurement results showed that the amount of compound 4 carried in the resulting carrier for solid-phase nucleic acid synthesis 5-2 was 37.5 µmol/g.

### [5] Chemical Structure of Carrier for Solid-Phase Nucleic Acid Synthesis as Comparative Example (Conventional Product)

| | |
|---|---|
| Chemical Structure of Glen UnySupport | Chemical Structure of Universal Support III |
| 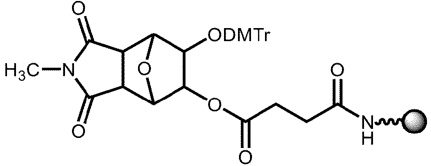 | 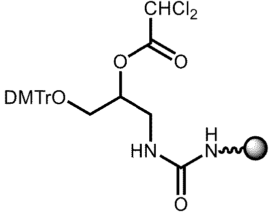 |

### [6] Synthesis of Nucleic Acid and Examination of Cleavage Efficiency

### DNA (5'-TTT TTT TTT T-3')

### Cleavage conditions: ammonia water, 15 hours (55°C)

**Table 3**

| Table 3 | | | |
|---|---|---|---|
| | | DNA remaining on solid-phase carrier after cleaving DNA (1 µmol synthesis) | Cleavage efficiency |
| Comp. Ex. 1 | Universal Support III | 0.342 µmol | 65.8% |
| Ex. 1 | Carrier for solid-phase nucleic acid synthesis 5-1 | 0.006 µmol | 99.4% |
| Ex. 2 | Carrier for solid-phase nucleic acid synthesis 5-2 | 0.001 µmol | 99.9% |

### RNA (5'-UGU GAC UGA CUG UGA CUG AC-3')

### Cleavage conditions: ammonia water/methylamine (v/v = 1/1), 90 minutes (room temperature) → 10 minutes (65°C)

**Table 4**

| Table 4 | | | |
|---|---|---|---|
| | | RNA remaining on solid-phase carrier after cleaving RNA (1 µmol synthesis) | Cleavage efficiency |
| Comp. Ex. 2 | Universal Support III | 0.411 µmol | 55.2% |
| Ex. 3 | Carrier for solid-phase nucleic acid synthesis 5-1 | 0.058 µmol | 91.6% |
| Ex. 4 | Carrier for solid-phase nucleic acid synthesis 5-2 | 0.067 µmol | 90.8% |

The results show that when the carrier for solid-phase nucleic acid synthesis 5-1 and carrier for solid-phase nucleic acid synthesis 5-2 of the present invention were used, higher cleavage efficiency (90% or more) was achieved for both DNA and RNA compared to when Universal Support III was used.

### [7] Nucleic Acid Crude Purity Analysis

### DNA (5'-TTT TTT TTT T-3')

**Table 5**

| Table 5 | | | |
|---|---|---|---|
| | | DNA (1 µmol synthesis) LC-MS purity (FLP) | LC-MS purity (linker adducts) |
| Comp. Ex. 3 | Glen UnySupport | 87 A% | ND |
| Ex. 5 | Carrier for solid-phase nucleic acid synthesis 5-1 | 81A% | ND |
| Ex. 6 | Carrier for solid-phase nucleic acid synthesis 5-2 | 81A% | ND |

### RNA (5'-UGU GAC UGA CUG UGA CUG AC-3')

**Table 6**

| Table 6 | | | |
|---|---|---|---|
| | | RNA (1 µmol synthesis) LC-MS purity (FLP) | LC-MS purity (linker adducts) |
| Comp. Ex. 4 | Glen UnySupport | 43 A% | 31A% |
| Ex. 7 | Carrier for solid-phase nucleic acid synthesis 5-1 | 69 A% | ND |
| Ex. 8 | Carrier for solid-phase nucleic acid synthesis 5-2 | 69 A% | ND |

The results confirmed that when the carrier for solid-phase nucleic acid synthesis 5-1 and carrier for solid-phase nucleic acid synthesis 5-2 of the present invention were used, no by-products (linker adducts) were produced even during RNA synthesis, and higher purity crude products were obtained compared to when the conventional product Glen UnySupport was used.

### Industrial Applicability

The present invention relates to a linker for nucleic acid synthesis and a carrier for solid-phase nucleic acid synthesis, both of which can suppress the production of by-products (linker adducts) that occur when using conventional universal supports.

The linker for nucleic acid synthesis and carrier for solid-phase nucleic acid synthesis of the present invention are characterized in that the solid-phase carrier and the linker for nucleic acid synthesis are bonded via a bond (e.g., an amide bond) that is not cleaved under cleavage conditions, such as in ammonia water. The linker for nucleic acid synthesis and carrier for solid-phase nucleic acid synthesis of the present invention do not produce linker adducts, and highly pure oligonucleic acids can be obtained by using the linker for nucleic acid synthesis and carrier for solid-phase nucleic acid synthesis of the present invention.

## Claims

1. A method for producing a linker precursor for nucleic acid synthesis represented by the following formula (2): 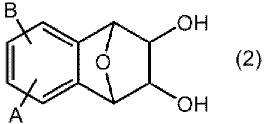 wherein
in formula (2), A is present at one substitutable position and represents a -CO-OR group, a -NH-CO-OR group, or a - CO-NH-CH₂-CO-OR group; and R represents a protecting group for carboxylic acids, and
in formula (2), B represents identically or differently, at a substitutable position, a hydrogen atom, a lower alkyl group, a lower alkoxy group, a cyano group, a nitro group, or a dialkylamino group;
the method comprising subjecting a compound represented by the following formula (1): 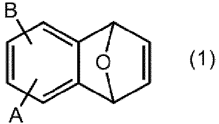
wherein in formula (1), A and B are the same as those in formula (2);
to an oxidation reaction in a solvent in the presence of an oxidizing agent and a reoxidizing agent.

2. The production method according to claim 1, wherein the oxidizing agent is at least one oxidizing agent selected from the group consisting of osmium tetroxide (OsO₄) and ozone (O₃).

3. The production method according to claim 1, wherein the reoxidizing agent is at least one reoxidizing agent selected from the group consisting of N-methylmorpholine-N-oxide (NMO), trimethylamine-N-oxide (Me₃NO), and tert-butyl hydroperoxide (TBHP).

4. The production method according to claim 1, wherein the solvent is a mixed solvent of water and a hydrophilic organic solvent.

5. A linker precursor for nucleic acid synthesis represented by the following formula (2): 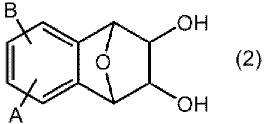 wherein
in formula (2), A is present at one substitutable position and represents a -CO-OR group, a -NH-CO-OR group, or a - CO-NH-CH₂-CO-OR group; and R represents a protecting group for carboxylic acids, and
in formula (2), B represents identically or differently, at a substitutable position, a hydrogen atom, a lower alkyl group, a lower alkoxy group, a cyano group, a nitro group, or a dialkylamino group.

6. A method for producing a linker precursor for nucleic acid synthesis represented by the following formula (3): 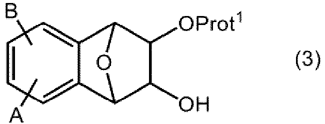 wherein
in formula (3), A is present at one substitutable position and represents a -CO-OR group, a -NH-CO-OR group, or a - CO-NH-CH₂-CO-OR group; and R represents a protecting group for carboxylic acids,
in formula (3), B represents identically or differently, at a substitutable position, a hydrogen atom, a lower alkyl group, a lower alkoxy group, a cyano group, a nitro group, or a dialkylamino group, and
in formula (3), Prot¹ represents a 4,4'-dimethoxytrityl group, a 4-methoxytrityl group, or a trityl group;
the method comprising reacting a compound represented by the following formula (2): 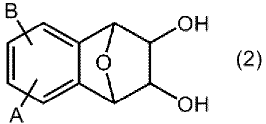
wherein in formula (2), A and B are the same as those in formula (3);
with 4,4'-dimethoxytrityl chloride (DMTr-Cl), 4-methoxytrityl chloride (MMTr-Cl), or trityl chloride (Tr-Cl) in a solvent.

7. The production method according to claim 6, wherein the solvent is at least one organic solvent selected from the group consisting of aprotic polar solvents.

8. A linker precursor for nucleic acid synthesis represented by the following formula (3): 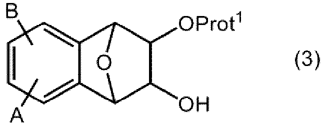 wherein
in formula (3), A is present at one substitutable position and represents a -CO-OR group, a -NH-CO-OR group, or a - CO-NH-CH₂-CO-OR group; and R represents a protecting group for carboxylic acids,
in formula (3), B represents identically or differently, at a substitutable position, a hydrogen atom, a lower alkyl group, a lower alkoxy group, a cyano group, a nitro group, or a dialkylamino group, and
in formula (3), Prot¹ represents a 4,4'-dimethoxytrityl group, a 4-methoxytrityl group, or a trityl group.

9. A method for producing a linker for nucleic acid synthesis represented by the following formula (4): 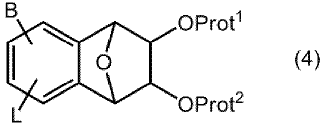 wherein
in formula (4), L is present at one substitutable position and represents a -CO-OH group, a -NH-CO-OH group, or a - CO-NH-CH₂-CO-OH group,
in formula (4), B represents identically or differently, at a substitutable position, a hydrogen atom, a lower alkyl group, a lower alkoxy group, a cyano group, a nitro group, or a dialkylamino group,
in formula (4), Prot¹ represents a 4,4'-dimethoxytrityl group, a 4-methoxytrityl group, or a trityl group, and
in formula (4), Prot² represents a protecting group for hydroxyl groups;
the method comprising subjecting a compound represented by the following formula (3): 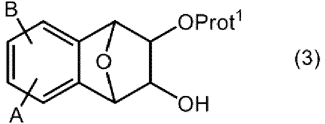 wherein
in formula (3), A is present at one substitutable position and represents a -CO-OR group, a -NH-CO-OR group, or a - CO-NH-CH₂-CO-OR group; and R represents a protecting group for carboxylic acids, and
in formula (3), B and Prot¹ are the same as those in formula (4);
to the following steps:
(1) performing hydrolysis in a solvent, and then
(2) protecting a hydroxyl group in a solvent.

10. The production method according to claim 9, wherein the solvent used in step (1) is at least one solvent selected from the group consisting of alkaline aqueous solutions, ether-based solvents, alcohol-based solvents, and halogen-based solvents.

11. The production method according to claim 9, wherein the solvent used in step (2) is at least one solvent selected from the group consisting of aprotic polar solvents and ether-based solvents.

12. A linker for nucleic acid synthesis represented by the following formula (4): 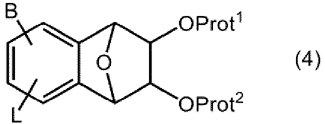 wherein
in formula (4), L is present at one substitutable position and represents a -CO-OH group, a -NH-CO-OH group, or a - CO-NH-CH₂-CO-OH group,
in formula (4), B represents identically or differently, at a substitutable position, a hydrogen atom, a lower alkyl group, a lower alkoxy group, a cyano group, a nitro group, or a dialkylamino group,
in formula (4), Prot¹ represents a 4,4'-dimethoxytrityl group, a 4-methoxytrityl group, or a trityl group, and
in formula (4), Prot² represents a protecting group for hydroxyl groups.

13. A method for producing a carrier for solid-phase nucleic acid synthesis represented by the following formula (5): 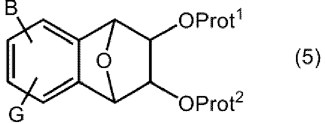 wherein
in formula (5), G is present at one substitutable position and represents the following formula (G1), (G2), or (G3),
in formula (G1), (G2), or (G3), SP represents a solid-phase carrier: 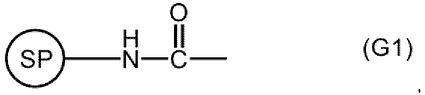 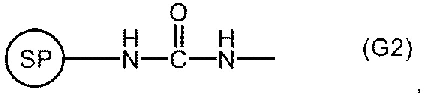 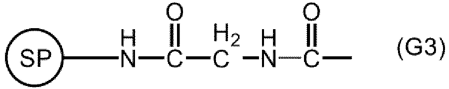
in formula (5), B represents identically or differently, at a substitutable position, a hydrogen atom, a lower alkyl group, a lower alkoxy group, a cyano group, a nitro group, or a dialkylamino group,
in formula (5), Prot¹ represents a 4,4'-dimethoxytrityl group, a 4-methoxytrityl group, or a trityl group, and
in formula (5), Prot² represents a protecting group for hydroxyl groups;
the method comprising reacting a compound represented by the following formula (4): 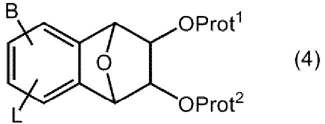 wherein
in formula (4), L is present at one substitutable position and represents a -CO-OH group, a -NH-CO-OH group, or a - CO-NH-CH₂-CO-OH group, and
in formula (4), B, Prot¹, and Prot² are the same as those in formula (5);
with a solid-phase carrier represented by the following formula: 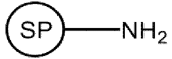
wherein SP represents a solid-phase carrier;
in a solvent in the presence of a coupling agent.

14. The production method according to claim 13, wherein the coupling agent is at least one coupling agent selected from the group consisting of 1-[bis(dimethylamino)methylene]-1H-1,2,3-triazolo[4,5-b]pyridinium 3-oxide hexafluorophosphate (HATU), 1-[bis(dimethylamino)methylene]-1H-benzotriazolium 3-oxide hexafluorophosphate (HBTU), O-(1H-6-chlorobenzotriazole-1-yl)-1,1,3,3-tetramethyluronium hexafluorophosphate (HCTU), 1-[bis(dimethylamino)methylene]-1H-1,2,3-triazolo[4,5-b]pyridinium 3-oxide tetrafluoroborate (TATU), 1-[bis(dimethylamino)methylene]-1H-benzotriazolium 3-oxide tetrafluoroborate (TBTU), N,N'-dicyclohexylcarbodiimide (DCC), 1-ethyl-3-(3-dimethylaminopropyl)carbodiimide hydrochloride (EDC-HCl), ethyl 2-cyano-2-((dimethyliminio) (morpholino)methyloxyimino)acetate hexafluorophosphate (COMU), diphenylphosphoryl azide (DPPA), 4-(4,6-dimethoxy-1,3,5-triazin-2-yl)-4-methylmorpholinium chloride (DMT-MM), benzotriazol-1-yl-oxy-tris(dimethylamino)-phosphonium hexafluorophosphate (BOP), and 1H-benzotriazol-1-yloxytripyrrolidinophosphonium hexafluorophosphate (PyBOP).

15. The production method according to claim 13, wherein the solvent is at least one organic solvent selected from the group consisting of aprotic polar solvents and halogen-based solvents.

16. A carrier for solid-phase nucleic acid synthesis represented by the following formula (5): 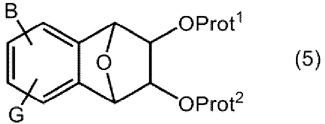 wherein
in formula (5), G is present at one substitutable position and represents the following formula (G1), (G2), or (G3),
in formula (G1), (G2), or (G3), SP represents a solid-phase carrier: 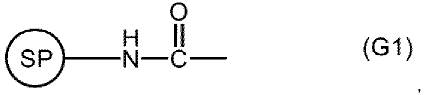 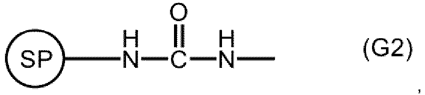 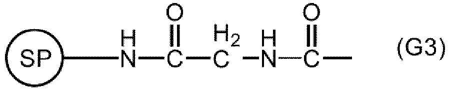
in formula (5), B represents identically or differently, at a substitutable position, a hydrogen atom, a lower alkyl group, a lower alkoxy group, a cyano group, a nitro group, or a dialkylamino group,
in formula (5), Prot¹ represents a 4,4'-dimethoxytrityl group, a 4-methoxytrityl group, or a trityl group, and
in formula (5), Prot² represents a protecting group for hydroxyl groups.
